# EUROPÄISCHE PATENTSCHRIFT

(11) **EP 0 995 749 B1**
(45) Veröffentlichungstag und Bekanntmachung des Hinweises auf die Patenterteilung: **07.03.2007**
(21) Anmeldenummer: 99120364.7
(22) Anmeldetag: 13.10.1999
(51) Int. Cl.: C07D 475/14

(54) **Reinigungs- und Kristallisationsverfahren für Riboflavin**
Process for purification and crystallisation of riboflavin
Procédé pour la purification et pour la crystallisation de riboflavine

(30) Priorität: 19.10.1998 EP 98119686
(43) Veröffentlichungstag der Anmeldung: 26.04.2000
(73) Patentinhaber: DSM IP Assets B.V., 6411 TE Heerlen (NL)
(72) Erfinder: Wagner, Gerhard, 79664 Wehr (DE)
(74) Vertreter: Müller, Ingrid

(56) Entgegenhaltungen:
- EP-A- 0 164 704
- EP-A- 0 307 767
- EP-A- 0 464 582
- EP-A- 0 730 034
- DE-A- 4 014 262
- US-A- 2 324 800
- US-A- 2 603 633
- US-A- 2 797 215
- CHEMICAL ABSTRACTS, vol. 113, no. 2, 9. Juli 1990 (1990-07-09) Columbus, Ohio, US; abstract no. 8823t, Seite 141; XP002125630 -& PATENT ABSTRACTS OF JAPAN vol. 14, no. 7 (C-673), 10. Januar 1990 (1990-01-10) & JP 01 254222 A (KUBOTA LTD), 11. Oktober 1989 (1989-10-11)

## Beschreibung

Die vorliegende Erfindung betrifft ein neues Verfahren zur Reinigung und Kristallisation von Riboflavin für Pharma- und Lebensmittelanwendungen.

Das zur Zeit im Handel befindliche Riboflavin wird teilweise synthetisch und teilweise biotechnologisch hergestellt, wobei die biotechnologischen Herstellungsverfahren in der letzten Zeit eindeutig auf dem Vormarsch sind. Gerade bei fermentativer Herstellungsweise ist die nahezu vollständige Reinigung und Aufkonzentrierung des Riboflavins, wie sie für Pharma- und Lebensmittelanwendungen erforderlich ist, ausgesprochen schwierig. Für solche Anwendungen wird Riboflavin in der Regel in saurem oder alkalischem Umfeld gelöst. Zellreste, Proteine, Peptide und Aminosäuren, die nach dem Lösen des Riboflavins selber je nach Substanz gelöst oder ungelöst vorliegen können, sind häufig nur mit relativ grossem Aufwand durch die Kombination mehrerer unterschiedlicher Einheitsoperationen abtrennbar. Das gelöste Riboflavin wird in der Regel durch unterschiedliche Verfahren meist oberhalb von 30°C auskristallisiert, und zwar als nadelförmige Kristalle, welche normalerweise der stabilen Modifikation A entsprechen (siehe beispielsweise die US-Patentschriften 2.324.800, 2.797.215 und 4.687.847). Ferner wird Riboflavin bisher ausschliesslich in der stabilen Kristallmodifikation A hergestellt und vertrieben. Da Riboflavin in dieser Form nur in sehr geringem Umfang wasserlöslich ist, ist das für Pharma- und für Lebensmittelanwendungen notwendige Löseverhalten relativ schlecht. Seit geraumer Zeit besteht daher der Wunsch, das Löseverhalten und damit auch die Bioverfügbarkeit von Riboflavin zu verbessern.

Verschiedene aus der Literatur bekannte Arbeiten betreffen unterschiedlich stabile Kristallmodifikationen von Riboflavin, die durch Ausfällung aus alkalischer Lösung gebildet werden; aus solchen Arbeiten ist allerdings bisher kein praktisches Betriebsverfahren entwickelt worden, was vermutlich auf den chemischen Abbau des Riboflavins in alkalischem Umfeld zurückzuführen ist (siehe beispielsweise die US-Patentschrift 2.603.633).

Das gegenwärtig vertriebene Riboflavin liegt teilweise in Form von sehr feinem Pulver, teilweise in Form von langen gelben Nadeln vor. Das feine Pulver staubt beträchtlich, weist eine ausgesprochen geringe Schüttdichte und ein schlechtes Fliessverhalten auf und lädt sich sehr leicht auf, so dass beispielsweise das Verpressen in Tabletten erschwert wird und Zusätze zur Verbesserung des Fliess- und Kompaktierverhaltens benötigt werden. Ebenso zeigen die Nadeln bei der Verarbeitung eine starke Staubentwicklung und sind problematisch bei der Weiterverarbeitung, wie beispielsweise bei der Mehlvitaminierung. Auch verschiedene bei der Kristallisation erfolgende Agglomerationsverfahren wurden bisher nicht zur grosstechnischen Herstellung von Riboflavin eingesetzt (siehe beispielsweise die kanadische Patentschrift 633.852 und die europäische Patentschrift 307.767). Weitere Agglomerationsverfahren erfolgen bei der Trocknung unter Verwendung von nadelförmigen Kristallen der Modifikation A (deutsche Offenlegungsschrift 4.014.262). Nach wie vor besteht der Wunsch, eine Form des Riboflavins herzustellen, welche wesentlich bessere physikalische Eigenschaften, wie beispielsweise Fliess- und Löseeigenschaften und Abriebsfestigkeit, besitzt und eine Reinheit (Gehalt an Riboflavin) von über 98% aufweist.

In der europäischen Patentpublikation 730.034 ist auf Seite 5, Zeilen 13-18, die Reinigung einer Lösung von. Riboflavin in Salzsäure mit Aktivkohle offenbart. Chem. Abs. 113, 8823t (1990) [JP 01/254222] offenbart die Verwendung von Querstromfiltration bei der Behandlung von wässrigen Lösungen oder von Abwasser zur Isolierung wertvoller Komponenten; die dabei verwendete Keramikmembran ist vorzugsweise im Voraus mit einer einen Filterhilfsstoff enthaltenen Aufschlämmung überzogen.

Ziel der vorliegenden Erfindung ist es, ausgehend von nadelförmigem Riboflavin, welches der stabilen Modifikation A entspricht und synthetisch oder biotechnologisch hergestellt worden ist, ein reineres Riboflavin mit über 98%igem Gehalt an Riboflavin herzustellen, welches deutlich bessere Löse- und Fliesseigenschaften besitzt als das gegenwärtig verfügbare Material. Im allgemeinen sollte das neue Riboflavin eine bessere Bioverfügbarkeit und bessere physikalische Eigenschaften, z.B. bei der Tablettierung, aufweisen.

Es ist nun eine relativ einfaches Verfahren gefunden worden, welches es erlaubt, ein derartiges Riboflavin bereitzustellen. Dieses Verfahren besteht im Grunde genommen aus einer sogenannten Vorreinigung und einer Kristallisation; der Kristallisation schliesst sich eine Trocknung an.

Es handelt sich bei dem erfindungsgemässen Verfahren um ein Verfahren zur Reinigung und Kristallisation von Riboflavin, das dadurch gekennzeichnet ist, dass man nadelförmiges Riboflavin der stabilen Modifikation A in einer wässrigen Mineralsäurelösung bei 5 bis 25°C unter intensiver Durchmischung auflöst, zur resultierenden Lösung Aktivkohle zugibt, nach Adsorption der gelösten Verunreinigungen aus der Lösung auf der Aktivkohle das die Aktivkohle enthaltende Medium einer Querstromfiltration über eine Keramikmembran mit einer Porengrösse von 20 bis 200 nm unterwirft, das resultierende Filtrat mit einer fünf- bis zehnfachen Menge (Vol. /Vol.) Wasser bei 4 bis 10°C versetzt, und die resultierenden ausgefallenen, sphärischen Kristalle von Riboflavin durch Zentrifugation oder Filtration abtrennt.

Nach Erhalt der Riboflavin-Kristalle auf diese Weise können die Kristalle gegebenenfalls mit Wasser gewaschen und anschliessend nach an sich bekannten Methoden getrocknet werden. Das so ergänzte Verfahren stellt einen weiteren Aspekt des oben definierten erfindungsgemässen Verfahrens dar.

Als Ausgangsmaterial des erfindungsgemässen Verfahrens wird nadelförmiges Riboflavin der Modifikation A eingesetzt, wie es beispielsweise bei der Produktion für Futtermittel anfällt. Dieses Riboflavin weist in der Regel einen Gehalt von etwa 85 bis zu etwa 98% und je nach Herstellungsweise chemische Nebenprodukte und/oder Fermentationsreste sowie Wasser auf, deren Gesamtmenge dementsprechend über 2 Gewichtsprozent beträgt.

Im ersten Verfahrensschritt wird die Ausgangsware trocken oder filterfeucht in der wässrigen Mineralsäurelösung gelöst. Darauf erfolgt die Auflösung durch eine Protonierungsreaktion. Beim Lösevorgang werden Fermentationsreste, wie Proteine, Peptide und Aminosäuren, und/oder chemische Nebenprodukte frei, die dann teilweise gelöst und teilweise als Feststoff vorliegen. Als Mineralsäure eignet sich insbesondere Salzsäure oder Salpetersäure, vorzugsweise die erstere, deren Konzentration im allgemeinen etwa 10% bis etwa 65% (Gewichtsprozent) beträgt.

Im Falle der bevorzugten wässrigen Salzsäurelösung liegt die Konzentration zweckmässigerweise im Bereich von etwa 18 bis etwa 24%. In einer solchen wässrigen Salzsäurelösung werden bis zu etwa 19% trockenes Riboflavin gelöst. Die Lösung ist damit nahezu gesättigt. Im allgemeinen richtet sich die Menge Riboflavin gegenüber der Menge wässriger Mineralsäure nach der Natur der Mineralsäure, der Konzentration der Lösung und der Lösetemperatur.

Zudem erfolgt die Auflösung des nadelförmigen Riboflavins in der wässrigen Mineralsäurelösung in der Regel bei 5 bis 25°C, vorzugsweise bei 10 bis 20° C, und zwar zweckmässigerweise unter intensiver Durchmischung, beispielsweise durch intensives Rühren.

Durch die Erhöhung der Temperatur und/oder der Durchmischung kann die Lösezeit reduziert werden. Je nach Temperatur und Durchmischungsgrad dauert der vollständige Lösevorgang in der Regel bis zu etwa 30 Minuten.

Als nächster Verfahrensschritt wird zur Lösung des Riboflavins in der wässrigen Mineralsäurelösung Aktivkohle zugegeben. Demzufolge werden die gelöst vorliegenden Verunreinigungen auf der Aktivkohle adsorbiert. Diese kann pulverisiert oder granuliert sein. Zweckmässigerweise werden zur adsorptiven Reinigung der gelösten Verunreinigungen aus der Lösung 0,5 bis 9% (Gewichtsprozent), vorzugsweise 3%, Aktivkohle bezogen auf den Riboflavingehalt zugegeben. Je nach Verunreinigung wird die Aktivkohle bis zu etwa 12 Stunden, vorzugsweise etwa 0,5 bis etwa 3 Stunden, in der Lösung belassen. Als Aktivkohle eignet sich sauer gewaschene Aktivkohle mit einer Schüttdichte von 250 bis 400 kg/m³, vorzugsweise 300 kg/m³, einer spezifischen Oberfläche von 1200 bis 1600 m²/g, vorzugsweise 1400 m²/g, und einer mittleren Partikelgrösse von 20 bis 70 µm. Beispiele geeigneter Aktivkohlen sind Norit^{®} CA1 und Bentonorit^{®} CA1, die sich besonders zur Adsorption von gelösten biologischen Verunreinigungen eignen, sowie Norit^{®} SX2, das sich seinerseits besonders zur Abtrennung von chemischen Verunreinigungen eignet.

Gewünschtenfalls kann der wässrigen Mineralsäurelösung neben Aktivkohle ein Filterhilfsmittel zugegeben werden, von dem zweckmässigerweise etwa 2 bis etwa 9 Gewichtsprozent bezogen auf den Riboflavingehalt eingesetzt werden. Als Filterhilfsmittel eignen sich beispielsweise Arbocel^{®} BWW 40 und B 800 der Firma Rettenmaier & Söhne GmbH + Co.

Die Abtrennung der Aktivkohle, des eventuell vorhandenen Filterhilfsmittels und der ungelöst vorliegenden Fermentationsreste erfolgt mittels der anschliessenden Querstromfiltration. Es wurde überraschenderweise gefunden, dass die Aktivkohle neben der Adsorption noch eine abrasive Wirkung auf die Deckschicht zeigt, die sich auf der Membran bildet. Durch diese Wirkung ist es erst möglich, die Membran über einen längeren Zeitraum stabil mit nahezu dem doppelten Durchsatz zu betreiben, als ohne Aktivkohle. Der Aktivkohle kommen also sowohl abrasive wie auch adsorptive Eigenschaften zu. Die Querstromfiltration erfolgt über eine Keramikmembran, welche eine Porengrösse von 20 bis 200 nm, vorzugsweise 50 nm, aufweist. Die im Kreislauf umgepumpte Aktivkohle bewirkt durch die Abrasion eine Reinigung der sich auf der Membran aufbauenden Deckschicht aus Kohle und Fermentationsresten. Die Querstromgeschwindigkeit über der Membran ist in der Regel relativ hoch; sie liegt zweckmässigerweise im Bereich von etwa 5 bis etwa 6 m/s. Um die Deckschicht nicht übermässig zu komprimieren, beträgt der transmembrane Druck zweckmässigerweise 1 bis 2 bar (0,1 bis 0,2 MPa).

Nach der Querstromfiltration wird die von nahezu allen Verunreinigungen, der Aktivkohle sowie eventuell vorhandenem Filterhilfsmittel befreite Lösung von Riboflavin zur Kristallisation gebracht, was durch Zugabe von einer fünf- bis zehnfachen Menge Wasser erfolgt. Die damit erfolgende Deprotonierung des in der wässrigen Mineralsäurelösung vorhandenen Riboflavins führt zu dessen Ausfällung.

Die Temperatur des Mediums, in dem die Kristallisation stattfindet, variiert je nach Herstellungsweise und Verunreinigungsgrad des Riboflavins im Bereich von 4 bis 10°C variiert werden. Bei fermentativem oder relativ sauberem Material bieten sich im allgemeinen Temperaturen unter 10°C an. Die Kristallisation kann batchweise oder kontinuierlich durchgeführt werden, vorzugsweise kontinuierlich. Als Kristaller können Kaskaden oder einzelne Kessel eingesetzt werden. Insbesondere bei einzelnen Kesseln empfiehlt es sich, an unterschiedlichen Stellen im Kessel einzuspeisen. Innerhalb des Kristallers muss auf jeden Fall eine sehr gute makroskopische Durchmischung eingestellt werden. Dies kann beispielsweise durch Einsatz eines zweistufigen Rührwerkes realisiert werden, wobei die Feedlösungen um 180° versetzt auf der oberen und unteren Rührerebene zugespeist werden. Dabei wird zweckmässigerweise auf der oberen Ebene das Wasser, auf der unteren die Mineralsäurelösung des Riboflavins zugegeben. Das Rühren soll ausgesprochen schonend erfolgen, um die Kristalle nicht zu zerstören. Die Verweilzeit beträgt geeigneterweise 5 bis 25 Minuten, vorzugsweise etwa 10 bis 13 Minuten. Die anschliessende Filtration erfolgt mit einem Filter oder einer Zentrifuge; vorzugsweise wird ein Bandfilter eingesetzt, auf dem auch die eventuell durchgeführte Wäsche sehr effizient ist. Die Trocknung kann auf an sich bekannte Weise durchgeführt werden.

Die anfangs relative Uebersättigung im Kristaller (vor Zugabe von Wasser) kann durch die Rückführung der Mutterlauge von der Wäsche mit dem den Kristaller zulaufenden Wasser eingestellt werden. Das Verhältnis Mutterlauge:Wasser beträgt zweckmässigerweise etwa 1:1 bis etwa 1:8. Die relative Uebersättigung kann über die im Kristaller vorliegende Leitfähigkeit beurteilt werden, wobei idealerweise ein Bereich von etwa 170 bis etwa 200 mS/cm eingehalten wird. Je nach Leitfähigkeit kann auf die Rückführung der Mutterlauge verzichtet werden. Im Falle der Rückführung wird sie vorzugsweise über die sich im Kristaller einstellende Leitfähigkeit geregelt.

Es hat sich nun überraschenderweise gezeigt, dass es durch eine geeignete Wahl der Mischungsverhältnisse, Temperaturen, Durchmischung und Verweilzeit möglich ist, bei dem Kristallisationsschritt des erfindungsgemässen Verfahrens eine instabilere Modifikation des Riboflavins zu kristallisieren, die sphärisch mit einer stacheligen Oberfläche ist und damit eine wesentlich grössere Oberfläche besitzt als die bekannten nadelförmigen Kristalle der Modifikation A. Ueberraschenderweise entsteht das sphärische Kristall nicht durch einen Agglomerationsvorgang, wie es bisher allgemein für sphärische Kristalle in der Literatur beschreiben wird [siehe beispielsweise die europäische Patentschrift 307.767 und Can. J. Chem. Eng. 47 (4), 166-170 (1969)], vielmehr wachsen bei dem neuen Verfahren aus einem zunächst auskristallisierten kleinen, wahrscheinlich amorphen Keim nadelförmige Kristalle heraus. Die so entstandenen dendritischen Kristalle entsprechen der besser löslichen Modifikation B bzw. C, die zum einen ausreichend lagerstabil sind und zum anderen durch die instabilere Modifikation und grössere Oberfläche herausragende Löseeigenschaften und infolge ihrer kugelförmigen Gestalt herausragende Fliesseigenschaften besitzen. Durch das erfindungsgemässe Verfahren werden zudem Riboflavin-Kristalle mit einer höheren Abriebfestigkeit als bei Agglomeraten erzeugt.

Wie oben erwähnt, wird das Kristallisat mittels Filtration oder Zentrifugation abgetrennt. Der Filterkuchen wird dann vorzugsweise mit Wasser gewaschen, wonach der feuchte Filterkuchen getrocknet werden kann.

Das erfindungsgemässe Verfahren wird durch die nachfolgenden Beispiele veranschaulicht.

### Beispiel 1

Als Ausgangsmaterial des nachfolgend beschriebenen Verfahrens wurde fermentativ hergestelltes Riboflavin verwendet, welches einen Gehalt an Riboflavin von 97,02% (nach HPLC), eine Restfeuchtigkeit (H₂O) von 0,80% sowie einen Gehalt an Aminosäuren von 1,11% aufwies und als nadelförmige Kristalle der stabilen Modifikation A vorlag.

350,0 g dieses Ausgangsmaterials wurden in 1708,6 g 24%iger Salzsäure bei 22°C unter Rühren aufgelöst. Nach etwa 15 - 20 Minuten Lösezeit lag eine braun-schwarze Lösung vor, die etwa 17% Riboflavin enthielt.

Man gab anschliessend zur Lösung 16 g (etwa 3% der Riboflavinmenge) Aktivkohle (Norit^{®} CA1) zu und liess das Gemisch noch 4 Stunden nachrühren. Das Gemisch wurde in den Doppelmantelfeedtank einer Labor-Membrananlage gefüllt. Der Tank wurde gekühlt, um eine Temperatur von maximal 35°C einhalten zu können. Mittels einer Zentrifugalpumpe wurde die Lösung über eine Keramikmembran mit einer effektiven Membranfläche von 0,0055 m² gepumpt. Der transmembrane Druck wurde auf 1,5 bar (0,15 MPa) eingeregelt, die Querstromgeschwindigkeit über die Membran auf 6 m/s. Es ergab sich ein Permeatdurchsatz von etwa 100 l/m²/h, der bis nahezu zum Ende der Filtration aufrecht gehalten werden konnte.

Die salzsaure Riboflavinlösung wurde dann in einem kontinuierlich betriebenen Fällungskristaller zur Ausfällung gebracht

Der 3 I-Fällungskristaller wurde zunächst mit etwa 2 l Wasser gefüllt und die Flüssigkeit mit einem zweistufigen Schrägblattrührer bei 100 UpM gerührt und anschliessend auf 10°C abgekühlt. Danach wurde gleichzeitig kontinuierlich 1590 g/h salzsaure Riboflavinlösung auf den oberen Rührer und etwa 9000 g/h Wasser auf den unteren Rührer bei etwa 10°C zudosiert. Etwa 2 - 4 Minuten nach dem Start begann das Riboflavin als orange-gelbe Kristalle auszukristallisieren. Zu Beginn sahen die gefällten Kristalle flockenartig aus, welche aber nach 20 - 30 Minuten in körnige Partikel übergingen. Die Kristallsuspension wurde dann kontinuierlich abgelassen, nachdem im Kristaller die 3 I-Marke (Doppelmantelende) erreicht worden war (d.h. nach etwa 7 Minuten). Das Ventil wurde so eingestellt, dass sich das Niveau bei der 3 l-Marke einpendelte. Die abfliessende Suspension wurde direkt auf eine P3-Nutsche gegeben und der Feststoff dort von der Lösung abgetrennt.

Nach jeweils 15 Minuten sammelte man etwa 2500 ml Suspension und erhielt einen Filterkuchen von etwa 1 cm Dicke. Dieser wurde dann mit 1300 ml Wasser portionenweise gewaschen, bis ein pH von etwa 5 erreicht wurde.

Das feuchte, gelbe Kristallisat (65 - 75% Restfeuchte) wurde anschliessend getrocknet.

### Beispiel 2

Wie in Beispiel 1 beschrieben, wurde eine Riboflavin-Lösung hergestellt und mit Aktivkohle versetzt. Im Gegensatz zu Beispiel 1 wurde die Lösung über eine Membran mit einer Porengrösse von etwa 50 nm gereinigt. Der transmembrane Druck lag bei 1,5 bis 1,7 bar (0,15 bis 0,17 MPa), die Querstromgeschwindigkeit bei 5 bis 6 m/s. Es ergab sich ein Permeatdurchsatz von etwa 70 l/m²/h. Die Kristallisation, Filtration und Wäsche erfolgten analog Beispiel 1. Die Kristallisationstemperatur lag zwischen 9 und 10°C, und die Trocknung erfolgte in einem Labortrockenschrank bei 100°C.

Die Ergebnisse der obigen zwei Beispiele sind in der nachfolgenden Tabelle zusammengefasst:

**Tabelle: Reinheiten und Eigenschaften des jeweils getrockneten Endproduktes**

| Beispiel | Modifikation (nach Röntgen-Strukturanalyse) | Riboflavin-Gehalt (nach HPLC) | Lumichrom-Gehalt (nach HPLC) | Lumiflavin-Gehalt (nach HPLC) | Amino säure-Gehalt |
|---|---|---|---|---|---|
| 1 | B | 98% | 0,08% | - | 0,1% |
| 2 | B | 98,9% | 0,15% | - | 0,06% |

Die jeweils fehlende Prozentzahl beinhaltet den Wassergehalt und sonstige geringe Verunreinigungen.

## Patentansprüche

1. Verfahren zur Reinigung und Kristallisation von Riboflavin, **dadurch gekennzeichnet, dass** man nadelförmiges Riboflavin der stabilen Modifikation A in einer wässrigen Mineralsäurelösung bei 5 bis 25°C unter intensiver Durchmischung auflöst, zur resultierenden Lösung Aktivkohle zugibt, nach Adsorption der gelösten Verunreinigungen aus der Lösung auf der Aktivkohle das die Aktivkohle enthaltende Medium einer Querstromfiltration über eine Keramikmembran mit einer Porengrösse von 20 bis 200 nm unterwirft, das resultierende Filtrat mit einer fünf- bis zehnfachen Menge (Vol. /Vol.) Wasser bei 4 bis 10°C versetzt, und die resultierenden ausgefallenen, sphärischen Kristalle von Riboflavin durch Zentrifugation oder Filtration abtrennt.

2. Verfahren nach Anspruch 1, **dadurch gekennzeichnet, dass** die so erhaltenen sphärischen Kristalle von Riboflavin mit Wasser gewaschen und anschliessend getrocknet werden.

3. Verfahren nach Anspruch 1 oder 2, **dadurch gekennzeichnet, dass** die Mineralsäure Salzsäure oder Salpetersäure, vorzugsweise Salzsäure, ist.

4. Verfahren nach einem der Ansprüche 1 bis 3, **dadurch gekennzeichnet, dass** die Auflösung des nadelförmigen Riboflavins in der wässrigen Mineralsäurelösung bei 10 bis 20°C, erfolgt.

5. Verfahren nach einem der Ansprüche 1 bis 4, **dadurch gekennzeichnet, dass** zur Lösung des nadelförmigen Riboflavins in der wässrigen Mineralsäurelösung 0,5 bis 9% (Gewichtsprozent) Aktivkohle bezogen auf den Riboflavingehalt zugegeben werden.

6. Verfahren nach einem der Ansprüche 1 bis 5, **dadurch gekennzeichnet, dass** als Aktivkohle sauer gewaschene Aktivkohle mit einer Schüttdichte von 250 bis 400 kg/m³, einer spezifischen Oberfläche von 1200 bis 1600 m²/g und einer mittleren Partikelgrösse von 20 bis 70 µm verwendet wird.

7. Verfahren nach einem der Ansprüche 1 bis 6, **dadurch gekennzeichnet, dass** neben Aktivkohle ein Filterhilfsmittel zugegeben wird.

8. Verfahren nach einem der Ansprüche 1 bis 7, **dadurch gekennzeichnet, dass** die Keramikmembran eine Porengrösse von etwa 50 nm aufweist.

9. Verfahren nach einem der Ansprüche 1 bis 8, **dadurch gekennzeichnet, dass** das Verfahren kontinuierlich durchgeführt wird, und die Verweilzeit im Kristaller bei der Kristallisation 5 bis 25 Minuten beträgt.

10. Verfahren nach einem der Ansprüche 1 bis 9, **dadurch gekennzeichnet, dass** die resultierenden ausgefallenen, sphärischen Kristalle von Riboflavin auf einem Bandfilter gesammelt, abgetrennt und getrocknet werden.

11. Verfahren nach Anspruch 5, **dadurch gekennzeichnet, dass** zur Lösung des nadelförmigen Riboflavins in der wässrigen Mineralsäurelösung etwa 3% Aktivkohle bezogen auf den Riboflavingehalt zugegeben werden.

12. Verfahren nach Anspruch 6, **dadurch gekennzeichnet, dass** als Aktivkohle sauer gewaschene Aktivkohle mit einer Schüttdichte von etwa 300 kg/m³ und einer spezifischen Oberfläche von etwa 1400 m²/g verwendet wird.

13. Verfahren nach Anspruch 9, **dadurch gekennzeichnet, dass** die Verweilzeit im Kristaller bei der Kristallisation 10 bis 13 Minuten beträgt.

## Claims

1. Process for the purification and crystallization of riboflavin, **characterized in that** needle-shaped riboflavin of the stable modification A is dissolved in an aqueous mineral acid solution at 5 to 25°C with intensive commixing, active charcoal is added to the resulting solution to adsorb dissolved impurities from the solution onto the active charcoal, the medium containing the active charcoal is subjected to a crossflow filtration over a ceramic membrane having a pore size of 20 to 200 nm, the resulting filtrate is admixed with five to ten times (vol./vol.) of water at 4 to 10°C, and the resulting precipitated, spherical crystals of riboflavin are separated off by centrifugation or filtration.

2. Process according to Claim 1, **characterized in that** the spherical crystals of riboflavin thus obtained are washed with water and subsequently dried.

3. Process according to Claim 1 or 2, **characterized in that** the mineral acid is hydrochloric acid or nitric acid, preferably hydrochloric acid.

4. Process according to any one of Claims 1 to 3, **characterized in that** the dissolving of the needle-shaped riboflavin in the aqueous mineral acid solution is effected at 10 to 20°C.

5. Process according to any one of Claims 1 to 4, **characterized in that** the amount of active charcoal added to the solution of the needle-shaped riboflavin in the aqueous mineral acid solution is 0.5% to 9% (weight per cent), based on the riboflavin content.

6. Process according to any one of Claims 1 to 5, **characterized in that** the active charcoal used is acid-washed active charcoal having a bulk density of 250 to 400 kg/m³, a specific surface area of 1200 to 1600 m²/g and an average particle size of 20 to 70 µm.

7. Process according to any one of Claims 1 to 6, **characterized in that** a filter aid is added as well as active charcoal.

8. Process according to any one of Claims 1 to 7, **characterized in that** the ceramic membrane has a pore size of about 50 nm.

9. Process according to any one of Claims 1 to 8, **characterized in that** the process is carried out continuously and the residence time in the crystallizer during the crystallization is 5 to 25 minutes.

10. Process according to any one of Claims 1 to 9, **characterized in that** the resulting precipitated, spherical crystals of riboflavin are collected, separated off and dried on a belt filter.

11. Process according to Claim 5, **characterized in that** the amount of active charcoal added to the solution of the needle-shaped riboflavin in the aqueous mineral acid solution is about 3%, based on the riboflavin content.

12. Process according to Claim 6, **characterized in that** the active charcoal used is acid-washed active charcoal having a bulk density of about 300 kg/m³ and a specific surface area of about 1400 m²/g.

13. Process according to Claim 9, **characterized in that** the residence time in the crystallizer during the crystallization is 10 to 13 minutes.

## Revendications

1. Procédé pour la purification et la cristallisation de riboflavine, **caractérisé en ce qu'**on dissout de la riboflavine en forme d'aiguilles de la modification stable A dans une solution aqueuse d'un acide minéral à 5 jusqu'à 25°C en mélangeant intensivement, on ajoute du charbon actif à la solution obtenue, on soumet, après l'adsorption des impuretés dissoutes de la solution sur le charbon actif, le milieu contenant le charbon actif à une filtration à flux transversal sur une membrane en céramique avec une grosseur de pores de 20 à 200 nm, on mélange le filtrat obtenu avec cinq à dix fois (V/V) la quantité d'eau à 4 jusqu'à 10°C et on sépare les cristaux sphériques précipités obtenus de riboflavine par centrifugation ou filtration.

2. Procédé selon la revendication 1, **caractérisé en ce que** les cristaux sphériques de riboflavine ainsi obtenus sont lavés avec de l'eau et ensuite séchés.

3. Procédé selon la revendication 1 ou 2, **caractérisé en ce que** l'acide minéral est l'acide chlorhydrique ou l'acide nitrique, de préférence l'acide chlorhydrique.

4. Procédé selon l'une quelconque des revendications 1 à 3, **caractérisé en ce que** la dissolution de la riboflavine en forme d'aiguilles dans la solution aqueuse d'acide minéral est réalisée à 10 jusqu'à 20°C.

5. Procédé selon l'une quelconque des revendications 1 à 4, **caractérisé en ce qu'**on ajoute à la solution de la riboflavine en forme d'aiguilles dans la solution aqueuse d'acide minéral 0,5 à 9% (en poids) de charbon actif par rapport à la teneur en riboflavine.

6. Procédé selon l'une quelconque des revendications 1 à 5, **caractérisé en ce qu'**on utilise comme charbon actif un charbon actif lavé à l'acide présentant une densité apparente de 250 à 400 kg/m³, une surface spécifique de 1 200 à 1 600 m²/g et une grosseur moyenne des particules de 20 à 70 µm.

7. Procédé selon l'une quelconque des revendications 1 à 6, **caractérisé en ce qu'**on ajoute, en plus du charbon actif, un adjuvant de filtration.

8. Procédé selon l'une quelconque des revendications 1 à 7, **caractérisé en ce que** la membrane céramique présente une taille de pores d'environ 50 nm.

9. Procédé selon l'une quelconque des revendications 1 à 8, **caractérisé en ce que** le procédé est réalisé en continu et le temps de séjour dans le cristalliseur lors de la cristallisation est de 5 à 25 minutes.

10. Procédé selon l'une quelconque des revendications 1 à 9, **caractérisé en ce que** les cristaux sphériques précipités obtenus de riboflavine sont rassemblés sur un filtre à bande, séparés et séchés.

11. Procédé selon la revendication 5, **caractérisé en ce qu'**on ajoute à la solution de la riboflavine en forme d'aiguilles dans la solution aqueuse d'acide minéral environ 3% de charbon actif par rapport à la teneur en riboflavine.

12. Procédé selon la revendication 6, **caractérisé en ce qu'**on utilise comme charbon actif un charbon actif lavé à l'acide présentant une densité apparente d'environ 300 kg/m³ et une surface spécifique d'environ 1 400 m²/g.

13. Procédé selon la revendication 9, **caractérisé en ce que** le temps de séjour dans le cristalliseur lors de la cristallisation est de 10 à 13 minutes.
